(19)

Europäisches
Patentamt
European
Patent Office
Office européen
des brevets

(11) **EP 3 133 057 A1**

(12) **EUROPEAN PATENT APPLICATION**
published in accordance with Art. 153(4) EPC

(43) Date of publication:
**22.02.2017 Bulletin 2017/08**

(21) Application number: **15780158.0**

(22) Date of filing: **14.04.2015**

(51) Int Cl.:
**C07C 51/60** *(2006.01)* **C07C 53/42** *(2006.01)*
**C07C 57/66** *(2006.01)*

(86) International application number:
**PCT/JP2015/061503**

(87) International publication number:
**WO 2015/159892 (22.10.2015 Gazette 2015/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **14.04.2014  JP 2014082538**

(71) Applicant: **NOF Corporation**
**Shibuya-ku**
**Tokyo 150-6019 (JP)**

(72) Inventors:
• **FUJITA Hiroya**
**Amagasaki-shi**
**Hyogo 660-0095 (JP)**

• **OHARA Shinji**
**Amagasaki-shi**
**Hyogo 660-0095 (JP)**
• **MARUYAMA Kei-ichi**
**Amagasaki-shi**
**Hyogo 660-0095 (JP)**
• **IWATA Tomoki**
**Amagasaki-shi**
**Hyogo 660-0095 (JP)**

(74) Representative: **Hoffmann Eitle**
**Patent- und Rechtsanwälte PartmbB**
**Arabellastraße 30**
**81925 München (DE)**

(54) **PRODUCTION METHOD FATTY ACID CHLORIDE AND FATTY ACID CHLORIDE**

(57) Provided is a fatty acid chloride which is manufactured by a simple method, which does not cloud and which has stable color over time. A fatty acid chloride is produced by causing a fatty acid with a carbon number of 8-22 and phosphorous trichloride in a proportion of one-third to two-thirds with respect to the fatty acid to react and removing phosphorous acid that appears as a byproduct to obtain a reaction product. Next, the reaction product is treated at a temperature of 10-60°C, a nitrogen flow rate of $1.0 \times 10^{-4}$ to $1.0 \times 10^{-2}$ m³/kg·hr, and a pressure of $133.3 \times 10^{-2}$ to $133.3 \times 10^{2}$ Pa, the unreacted phosphorous trichloride is caused to react with the fatty acid and the fatty acid chloride to produce an organic phosphorous compound, and the unreacted phosphorous trichloride is distilled to obtain the fatty acid chloride.

**EP 3 133 057 A1**

**Description**

TECHNICAL FIELD

The present invention relates to a production method of a fatty acid chloride by producing using phosphorus trichloride and a fatty acid, and a fatty acid chloride obtained.

BACKGROUND ART

A fatty acid chloride is used in the synthesis of an alkyl ketene dimer, an organic peroxide, a surfactant, an intermediate of medicine or the like. In general, the fatty acid chloride can be obtained by reacting a fatty acid with a chlorinating agent. As the chlorinating agent, for example, phosphorus trichloride or carbonyl chloride is used.
The production method of a fatty acid chloride using phosphorus trichloride has an advantage in that the production is relatively simple, but on the other hand it has a disadvantage in that a phosphorus compound, for example, phosphorous acid by-produced in the reaction or unreacted phosphorus trichloride, or an unreacted fatty acid are present in the fatty acid chloride as impurities.
In order to solve the problem of impurities, a method of removing the phosphorus compound and fatty acid by distillation has been taken, but there is a problem in that the fatty acid chloride distilled is colored with the lapse of time.
In Patent Document 1 (JP-A-11-255703), a production method of a fatty acid chloride in which phosphorus trichloride is reduced by adding an additive capable of forming a complex with phosphorus trichloride and distilling the fatty acid chloride is described.
Also, in Patent Document 2 (JP-A-6-41000), a production method for obtaining an aliphatic carboxylic acid chloride containing no phosphorus component by adding a metal halide to purify is described. These techniques obtain a fatty acid chloride having stable hue by conducting purification using an additive to eliminate the phosphorus component from the fatty acid chloride.

PRIOR ART DOCUMENT

PATENT DOCUMENT

   Patent Document 1: JP-A-11-255703
   Patent Document 2: JP-A-6-41000

SUMMARY OF THE INVENTION

PROBLEMS THAT THE INVENTION IS TO SOLVE

As a result of the intensive investigations, the inventors have found that a fatty acid chloride which has stable hue with the lapse of time and is free from turbidity can be obtained by performing a prescribed simple operation to leave a phosphorus component in a prescribed amount.
A problem of the invention is to provide a fatty acid chloride which is free from turbidity and has stable hue with the lapse of time by a simple production method.

MEANS FOR SOLVING THE PROBLEMS

As a result of the intensive investigations, the inventors have found that a fatty acid chloride which is produced according to the steps described below is free from turbidity and has stable hue with the lapse of time to complete the invention.
Specifically, the invention includes the following items.

   (1) A production method of a fatty acid chloride comprising performing Step 1 and Step 2 in this order:

   Step 1: a step of reacting a fatty acid having from 8 to 22 carbon atoms with from 1/3 to 2/3 equivalent of phosphorus trichloride relative to the fatty acid to prepare a fatty acid chloride, and removing phosphorous acid which is a by-product to obtain a reaction product;
   Step 2: a step of treating the reaction product obtained in Step 1 at a temperature from 10 to 60°C, at a nitrogen flow rate from $1.0 \times 10^{-4}$ to $1.0 \times 10^{-2}$ m$^3$/kg·hr, and under a pressure from $133.3 \times 10^{-2}$ to $133.3 \times 10^2$ Pa so as to react an unreacted phosphorus trichloride with the fatty acid and the fatty acid chloride to prepare an organic phosphorus compound and so as to be distilled off the unreacted phosphorus trichloride, thereby obtaining a fatty acid chloride.

(2) The method described in (1), wherein a phosphorus content of an inorganic phosphorus compound contained in the fatty acid chloride obtained in Step 2 is from 0.03 to 0.3% by weight, and a phosphorus content of the organic phosphorus compound contained in the fatty acid chloride obtained in Step 2 is from 0.04 to 0.1% by weight.
(3) A fatty acid chloride obtained by the method described in (1) or (2).

ADVANTAGE OF THE INVENTION

According to the invention, a fatty acid chloride which is free from turbidity and has stable hue with the lapse of time can be obtained by a simple production method.

MODE FOR CARRYING OUT THE INVENTION

The invention will be described in detail hereinafter.
The invention relates to a production method of a fatty acid chloride comprising performing Step 1 and Step 2 in this order. Each of the steps is described below.

<Step 1>

Step 1 is a step of reacting a fatty acid having from 8 to 22 carbon atoms with from 1/3 to 2/3 equivalent of phosphorus trichloride relative to the fatty acid to prepare a fatty acid chloride, and removing phosphorous acid which is a by-product to obtain a reaction product.
The fatty acid used in the invention is a saturated or unsaturated fatty acid having from 8 to 22 carbon atoms. As specific examples, a fatty acid having a single composition, for example, lauric acid, myristic acid, palmitic acid, isopalmitic acid, stearic acid, isostearic acid, oleic acid or behenic acid, and a fatty acid having a mixed composition, for example, palm oil fatty acid, palm nucleus fatty acid or beef tallow fatty acid can be used. Lauric acid, myristic acid, palm oil fatty acid and palm nucleus fatty acid are preferred, and lauric acid and palm oil fatty acid are particularly preferred.
The content of phosphorus trichloride is from 1/3 to 2/3 equivalent in an equivalent ratio of phosphorus trichloride/fatty acid. The equivalent ratio of phosphorus trichloride/fatty acid is preferably from 1.1/3 to 1.8/3 equivalent, and more preferably from 1.3/3 to 1.7/3 equivalent.
The reaction temperature between phosphorus trichloride and a fatty acid is preferably from 40 to 90°C, more preferably from 45 to 80°C, and still more preferably from 50 to 70°C. The addition of phosphorus trichloride is preferably conducted stepwise and more preferably conducted dropwise. After the pouring of phosphorus trichloride, ripening is performed preferably for 0.5 to 5 hours, more preferably for 1 to 4 hours, and still more preferably for 1 to 3 hours.
The reaction product obtained is subjected to still standing to separate layers, and a lower layer (aqueous layer) is separated, whereby phosphorous acid which is soluble in the aqueous layer is removed. The time for the still standing to separate layers is preferably from 2 to 12 hours, more preferably from 3 to 11 hours, and most preferably from 4 to 10 hours. The temperature for the still standing to separate layers is preferably from 40 to 90°C, more preferably from 45 to 80°C, and most preferably from 50 to 70°C.

<Step 2>

Step 2 is a step of treating the reaction product obtained in Step 1 at a temperature from 10 to 60°C, at a nitrogen flow rate from $1.0 \times 10^{-4}$ to $1.0 \times 10^{-2}$ m$^3$/kg·hr, and under a pressure from $133.3 \times 10^{-2}$ to $133.3 \times 10^2$ Pa so as to react an unreacted phosphorus trichloride with the fatty acid and the fatty acid chloride to prepare an organic phosphorus compound and so as to be distilled off a part of the unreacted phosphorus trichloride.
Thus, it is possible that a phosphorus content of an inorganic phosphorus compound contained in the product obtained is set from 0.03 to 0.3% by weight and a phosphorus content of the organic phosphorus compound contained in the product obtained is set from 0.04 to 0.1% by weight.
As to the fatty acid chloride (organic layer after layer separation) before performing Step 2, the phosphorus content of the inorganic phosphorus compound is preferably from 0.3 to 2.0% by weight, more preferably from 0.5 to 1.7% by weight, and particularly preferably from 0.7 to 1.5% by weight, relative to the fatty acid chloride.
As to the fatty acid chloride (organic layer after layer separation) before performing Step 2, by setting the phosphorus content of the inorganic phosphorus compound to 0.3% by weight or more, the organic phosphorus compound is easily prepared in a sufficient amount and the unreacted fatty acid is easily reduced. Also, by setting the content to 2.0% by weight or less, a time necessary for removing the unreacted phosphorus trichloride can be reduced and the deterioration in hue of the fatty acid chloride can be inhibited.

The phosphorus content of the inorganic phosphorus compound contained in the fatty acid chloride obtained by performing Step 2 is preferably from 0.03 to 0.3% by weight, more preferably from 0.07 to 0.25% by weight, and still more preferably from 0.10 to 0.20% by weight. The phosphorus content exceeding 0.3% by weight may become a factor for causing white turbidity of the fatty acid chloride, whereas when the content is less than 0.03% by weight, the effect for stabilizing hue with the lapse of time may become poor.

Also, the phosphorus content of the organic phosphorus compound contained in the fatty acid chloride obtained by performing Step 2 is preferably from 0.04 to 0.1% by weight, more preferably from 0.05 to 0.09% by weight, and still more preferably from 0.06 to 0.08% by weight. The phosphorus content exceeding 0.1% by weight may cause turbidity or purity degradation of the fatty acid chloride, whereas when the content is less than 0.04% by weight, the effect for stabilizing hue with the lapse of time may become poor.

The content of the unreacted fatty acid contained in the fatty acid chloride obtained by performing Step 1 is from 1.0 to 5.0% by weight, and when the fatty acid chloride is treated according to Step 2, the content of the unreacted fatty acid is from 0.5 to 2.0% by weight. It is preferably from 0.5 to 1.5% by weight.

The temperature of Step 2 is from 10 to 60°C. When the temperature is lower than 10°C, there is a danger that preparation of the organic phosphorus compound in a sufficient amount becomes difficult. From this standpoint, the temperature of Step 2 is preferably 15°C or more, and more preferably 20°C or more. Also, when the temperature of Step 2 exceeds 60°C, hue of the fatty acid chloride deteriorates. From this standpoint, the temperature of Step 2 is preferably 50°C or less, and more preferably 40°C or less.

The nitrogen injection amount in Step 2 is from $1.0 \times 10^{-4}$ to $1.0 \times 10^{-2}$ m$^3$/kg·hr, preferably from $1.0 \times 10^{-3}$ to $7.0 \times 10^{-3}$ m$^3$/kg·hr, and more preferably from $3.0 \times 10^{-3}$ to $5.0 \times 10^{-3}$ m$^3$/kg·hr. When the amount is less than $1.0 \times 10^{-4}$ m$^3$/kg·hr, a time necessary for removing the unreacted phosphorus trichloride increases and there is a danger that hue of the fatty acid chloride deteriorates. When the amount exceeds $1.0 \times 10^{-2}$ m$^3$/kg·hr, there is a danger that the yield decreases due to entrainment.

The pressure of Step 2 is from $133.3 \times 10^{-2}$ to $133.3 \times 10^2$ Pa, preferably from $133.3 \times 10^{-1}$ to $66.7 \times 10^2$ Pa, and more preferably from $133.3$ to $26.7 \times 10^2$ Pa. When the pressure exceeds $133.3 \times 10^2$ Pa, a time necessary for removing the unreacted phosphorus trichloride increases and there is a danger that hue of the fatty acid chloride deteriorates. When the pressure is less than $133.3 \times 10^{-2}$, the amount of fatty acid chloride distilled off is likely increased and it is not effective.

The time for Step 2 is ordinarily from 1 to 13 hours, so that the phosphorus content of the organic phosphorus compound and the phosphorus compound content of the inorganic phosphorus compound contained in the fatty acid chloride can be adjusted to the desired values.

The inorganic phosphorus compound contained in the fatty acid chloride denotes a phosphorus compound which is distributed in a saturated saline solution layer in an oil-water separation operation using ethyl ether and a saturated saline solution, and includes, for example, phosphorus trichloride, phosphorous acid or a reaction product thereof. The phosphorus content of the inorganic phosphorus compound contained in the fatty acid chloride denotes a phosphorus content contained in the phosphorus compound which is distributed in the saturated saline solution layer in the oil-water separation operation using ethyl ether and a saturated saline solution.

The organic phosphorus compound contained in the fatty acid chloride denotes a phosphorus compound which is distributed in an ethyl ether layer in the oil-water separation operation using ethyl ether and a saturated saline solution, and is mainly composed of a reaction product of an inorganic phosphorus compound, for example, phosphorus trichloride, with the fatty acid and the fatty acid chloride. The phosphorus content of the organic phosphorus compound contained in the fatty acid chloride denotes a phosphorus content contained in the phosphorus compound which is distributed in the ethyl ether layer in the oil-water separation operation using ethyl ether and a saturated saline solution.

EXAMPLES

(Measuring method of phosphorus content)

Preparation of calibration curve

As a standard sample, an aqueous solution corresponding to 2 μg/ml of phosphorus was prepared using monopotassium phosphate (special grade). The aqueous solution was aliquoted in a separatory funnel in appropriate amounts (several kinds of amounts between 0 to 70 μg) by a whole pipet, and the total volume was made up to 50 ml with water. 15 ml of a 10% aqueous nitric acid solution, 5 ml of a 5% ammonium molybdate solution and 10 ml of n-butyl acetate were added thereto, and the mixture was shaken for 3 minutes and then stood still. The lower layer was aliquoted in another separatory funnel, 10 ml of n-butyl acetate was added thereto, and the mixture was shaken for 3 minutes and then stood still. The n-butyl acetate layer in the separatory funnel was transferred to a 50 ml measuring flask. 2 ml of 3% stannous chloride solution was added thereto, and the volume was fixed with ethyl alcohol. Using a spectral photometer, the absorbance at 725 nm was measured (10 mm glass cell).

Pre-treatment method

To a separatory funnel (A) were added 50 ml of a saturated saline solution and 20 ml of ethyl ether, and from 0.1 to 0.5 g of a fatty acid chloride was weighed and added thereto. The separatory funnel (A) was shaken for 3 minutes and then stood still to separate layers. The saturated saline solution layer of the lower layer separated was aliquoted in another separatory funnel (B). To the separatory funnel (B) was added 20 ml of ethyl ether, and the separatory funnel (B) was shaken for 3 minutes and then stood still to separate layers. Also, to the separatory funnel (A) containing the remaining ethyl ether layer was added 25 ml of a saturated saline solution, and the separatory funnel (A) was shaken for 3 minutes and then stood still to separate layers. Then, the lower layers of the separatory funnel (A) and the separatory funnel (B) were aliquoted in the same conical beaker (C) to obtain the saturated saline solution layer. Also, the solutions remained in the separatory funnel (A) and the separatory funnel (B) were aliquoted in the same Kjeldahl flask (D) to obtain the ethyl ether layer.

Measuring method of phosphorus content of inorganic phosphorus compound

To the saturated saline solution layer in the conical beaker (C) were added 1 ml of a 10% aqueous nitric acid solution and 5 ml of a 2% potassium permanganate solution, and the mixture was heated at 200°C to oxidized phosphorus contained. After preparation of brown precipitates of manganese oxide oxidized, the heating was continued for about 10 minutes, and then a 10% sodium sulfite solution was dropwise added thereto to reduce. After cooling to room temperature, a few drops of bromphenol blue indicator were added, and neutralized with a 14% aqueous ammonia. The solution was transferred to a 200 ml measuring flask, and the volume was fixed with water. 50 ml of the solution was aliquoted in a separatory funnel by a whole pipet. 15 ml of a 10% aqueous nitric acid solution, 5 ml of a 5% ammonium molybdate solution and 10 ml of n-butyl acetate were added thereto, and the mixture was shaken for 3 minutes and then stood still. The lower layer was aliquoted in another separatory funnel, 10 ml of n-butyl acetate was added thereto, and the mixture was shaken for 3 minutes and then stood still. The n-butyl acetate layer in the separatory funnel was transferred to a 50 ml measuring flask. 2 ml of a 3% stannous chloride solution was added thereto, and the volume was fixed with ethyl alcohol. Using a spectral photometer, the absorbance at 725 nm was measured (10 mm glass cell). The phosphorus content was obtained from the calibration curve previously prepared.
Also, a blank experiment was performed in parallel with the experiment.

Measuring method of phosphorus content of organic phosphorus compound

The ethyl ether in the Kjeldahl flask (D) was completely distilled off. 5 ml of sulfuric acid was added thereto, and carbonization was conducted by a Kjeldahl cracking unit. After cooling the inside of the flask to room temperature, about 5 ml of a hydrogen peroxide solution was gradually added thereto from a dropping funnel, and decomposition was conducted by a Kjeldahl cracking unit. Then, about 15 ml of a hydrogen peroxide solution was continuously added dropwise from a dropping funnel at a rate of about 1.5 ml per minute. The solution was concentrated to expel almost the hydrogen peroxide solution, and after generating white smoke of sulfuric acid, the solution turned colorless and transparent. After allowing to cool to room temperature, 50 ml of water and 1 ml of a 2% potassium permanganate solution were added thereto, decomposition of the hydrogen peroxide and oxidation were performed at the same time in the Kjeldahl cracking unit to prepare brown precipitates of manganese oxide, then the heating was continued for about 10 minutes, and then a 10% sodium sulfite solution was dropwise added thereto to reduce. After cooling to room temperature, a few drops of bromphenol blue indicator were added, and neutralized with a 14% aqueous ammonia. The solution was transferred to a 200 ml measuring flask, and the volume was fixed with water. 50 ml of the solution was aliquoted in a separatory funnel by a whole pipet. 15 ml of a 10% aqueous nitric acid solution, 5 ml of a 5% ammonium molybdate solution and 10 ml of n-butyl acetate were added thereto, and the mixture was shaken for 3 minutes and then stood still. The lower layer was aliquoted in another separatory funnel, 10 ml of n-butyl acetate was added thereto, and the mixture was shaken for 3 minutes and then stood still. The n-butyl acetate layer in the separatory funnel was transferred to a 50 ml measuring flask. 2 ml of a 3% stannous chloride solution was added thereto, and the volume was fixed with ethyl alcohol. Using a spectral photometer, the absorbance at 725 nm was measured (10 mm glass cell). The phosphorus content was obtained from the calibration curve previously prepared.
Also, a blank experiment was performed in parallel with the experiment.

$$\text{Phosphorus content (\% by weight) of inorganic phosphorus compound =}$$

$$\text{(Phosphorus content (g) obtained from calibration curve/Sample collection quantity (g))} \times \text{Distillation rate} \times 100$$

$$\text{Phosphorus content (\% by weight) of organic phosphorus compound =}$$

$$\text{(Phosphorus content (g) obtained from calibration curve/Sample collection quantity (g))} \times \text{Distillation rate} \times 100$$

(Example 1: Palm oil fatty acid chloride)

To palm oil fatty acid (400.0 g) was added dropwise 1.5/3 equivalent of phosphorus trichloride (130.0 g) at from 50 to 60°C to perform a chlorination reaction. After standing still for 2 hours to separate layers, phosphorous acid (57.0 g) of the lower layer was removed to obtain a reaction solution (473.0 g). In the reaction solution after removing the phosphorous acid, the phosphorus content of the organic phosphorus compound was 0.02% by weight, and the phosphorus content of the inorganic phosphorus compound was 0.90% by weight.

Thereafter, the reaction solution was treated at 30°C, at a nitrogen flow rate of $3.0 \times 10^{-3}$ m$^3$/kg·hr, and under a pressure of 665 Pa for 2 hours to remove the unreacted phosphorus trichloride, thereby obtaining the desired palm oil fatty acid chloride (452.6 g). In the palm oil fatty acid chloride obtained, the phosphorus content of the organic phosphorus compound was 0.06% by weight, and the phosphorus content of the inorganic phosphorus compound was 0.15% by weight.

(Example 2: Lauric acid chloride)

To lauric acid (400.0 g) was added dropwise 1.5/3 equivalent of phosphorus trichloride (130.7 g) at from 50 to 60°C to perform a chlorination reaction. After standing still for 2 hours to separate layers, phosphorous acid (58.2 g) of the lower layer was removed to obtain a reaction solution (472.5 g). In the reaction solution after removing the phosphorous acid, the phosphorus content of the organic phosphorus compound was 0.02% by weight, and the phosphorus content of the inorganic phosphorus compound was 1.00% by weight.

Thereafter, the reaction solution was treated at 60°C, at a nitrogen flow rate of $3.0 \times 10^{-3}$ m$^3$/kg·hr, and under a pressure of $133.3 \times 10$ Pa for 2 hours to remove the unreacted phosphorus trichloride, thereby obtaining the desired lauric acid chloride (452.5 g). In the lauric acid chloride obtained, the phosphorus content of the organic phosphorus compound was 0.10% by weight, and the phosphorus content of the inorganic phosphorus compound was 0.10% by weight.

(Example 3: Palm oil fatty acid chloride)

To palm oil fatty acid (400.0 g) was added dropwise 1.8/3 equivalent of phosphorus trichloride (156.0 g) at from 50 to 60°C to perform a chlorination reaction. After standing still for 2 hours to separate layers, phosphorous acid (57.1 g) of the lower layer was removed to obtain a reaction solution (498.9 g). In the reaction solution after removing the phosphorous acid, the phosphorus content of the organic phosphorus compound was 0.04% by weight, and the phosphorus content of the inorganic phosphorus compound was 1.50% by weight.

Thereafter, the reaction solution was treated at 15°C, at a nitrogen flow rate of $3.0 \times 10^{-3}$ m$^3$/kg·hr, and under a pressure of $133.3 \times 10$ Pa for 4 hours to remove the unreacted phosphorus trichloride, thereby obtaining the desired palm oil fatty acid chloride (476.9 g). In the palm oil fatty acid chloride obtained, the phosphorus content of the organic phosphorus compound was 0.10% by weight, and the phosphorus content of the inorganic phosphorus compound was 0.30% by weight.

(Example 4: Stearic acid chloride)

To stearic acid (435.0 g) was added dropwise 2.0/3 equivalent of phosphorus trichloride (131.0 g) at from 60 to 65°C to perform a chlorination reaction. After standing still for 2 hours to separate layers, phosphorous acid (45.0 g) of the lower layer was removed to obtain a reaction solution (521.0 g). In the reaction solution after removing the phosphorous acid, the phosphorus content of the organic phosphorus compound was 0.05% by weight, and the phosphorus content of the inorganic phosphorus compound was 1.8% by weight.

Thereafter, the reaction solution was treated at 40°C, at a nitrogen flow rate of $3.0 \times 10^{-3}$ m$^3$/kg·hr, and under a pressure of 133.3 Pa for 2 hours to remove the unreacted phosphorus trichloride, thereby obtaining the desired stearic acid chloride (445.0 g). In the stearic acid chloride obtained, the phosphorus content of the organic phosphorus compound was 0.08% by weight, and the phosphorus content of the inorganic phosphorus compound was 0.11% by weight.

(Example 5: Palm oil fatty acid chloride)

To palm oil fatty acid (400 g) was added dropwise 1.3/3 equivalent of phosphorus trichloride (112.0 g) at from 50 to 60°C to perform a chlorination reaction. After standing still for 2 hours to separate layers, phosphorous acid (56. 6 g) of the lower layer was removed to obtain a reaction solution (455.4 g). In the reaction solution after removing the phosphorous acid, the phosphorus content of the organic phosphorus compound was 0.02% by weight, and the phosphorus content of the inorganic phosphorus compound was 0.62% by weight.

Thereafter, the reaction solution was treated at 30°C, at a nitrogen flow rate of $3.0 \times 10^{-3}$ m$^3$/kg·hr, and under a pressure of 665 Pa for 2 hours to remove the unreacted phosphorus trichloride, thereby obtaining the desired palm oil fatty acid chloride (432. 6 g). In the palm oil fatty acid chloride obtained, the phosphorus content of the organic phosphorus compound was 0.05% by weight, and the phosphorus content of the inorganic phosphorus compound was 0.11% by weight.

(Example 6: Lauric acid chloride)

To lauric acid (400.0 g) was added dropwise 1.5/3 equivalent of phosphorus trichloride (130.7 g) at from 50 to 60°C to perform a chlorination reaction. After standing still for 2 hours to separate layers, phosphorous acid (58.2 g) of the lower layer was removed to obtain a reaction solution (472.5 g). In the reaction solution after removing the phosphorous acid, the phosphorus content of the organic phosphorus compound was 0.02% by weight, and the phosphorus content of the inorganic phosphorus compound was 1.00% by weight.

Thereafter, the reaction solution was treated at 60°C, at a nitrogen flow rate of $8.0 \times 10^{-3}$ m$^3$/kg·hr, and under a pressure of $133.3 \times 10$ Pa for 1.5 hours to remove the unreacted phosphorus trichloride, thereby obtaining the desired lauric acid chloride (448.5 g). In the lauric acid chloride obtained, the phosphorus content of the organic phosphorus compound was 0.08% by weight, and the phosphorus content of the inorganic phosphorus compound was 0.08% by weight.

(Example 7: Stearic acid chloride)

To stearic acid (435.0 g) was added dropwise 2.0/3 equivalent of phosphorus trichloride (131.0 g) at from 60 to 65°C to perform a chlorination reaction. After standing still for 2 hours to separate layers, phosphorous acid (45.0 g) of the lower layer was removed to obtain a reaction solution (521.0 g). In the reaction solution after removing the phosphorous acid, the phosphorus content of the organic phosphorus compound was 0.05% by weight, and the phosphorus content of the inorganic phosphorus compound was 1.8% by weight.

Thereafter, the reaction solution was treated at 40°C, at a nitrogen flow rate of $3.0 \times 10^{-3}$ m$^3$/kg·hr, and under a pressure of $133.3 \times 10^{-1}$ Pa for 2 hours to remove the unreacted phosphorus trichloride, thereby obtaining the desired stearic acid chloride (443.0 g). In the stearic acid chloride obtained, the phosphorus content of the organic phosphorus compound was 0.07% by weight, and the phosphorus content of the inorganic phosphorus compound was 0.08% by weight.

(Comparative Example 1: Palm oil fatty acid chloride)

To palm oil fatty acid (400.0 g) was added dropwise 1.5/3 equivalent of phosphorus trichloride (130.0 g) at from 50 to 60°C to perform a chlorination reaction. After standing still for 2 hours to separate layers, phosphorous acid (57.5 g) of the lower layer was removed to obtain a reaction solution (472.5 g). In the reaction solution after removing the phosphorous acid, the phosphorus content of the organic phosphorus compound was 0.02% by weight, and the phosphorus content of the inorganic phosphorus compound was 0.9% by weight.

Thereafter, the reaction solution was distilled by a thin film distiller to obtain distilled palm oil fatty acid chloride (452.4 g). In the palm oil fatty acid chloride obtained, the phosphorus content of the organic phosphorus compound was 0.01% by weight, and the phosphorus content of the inorganic phosphorus compound was N. D. (less than 0.01% by weight).

(Comparative Example 2: Lauric acid chloride)

To lauric acid (400.0 g) was added dropwise 2.5/3 equivalent of phosphorus trichloride (218.0 g) at from 50 to 60°C to perform a chlorination reaction. After standing still for 2 hours to separate layers, phosphorous acid (58.2 g) of the lower layer was removed to obtain a reaction solution (558.6 g). In the reaction solution after removing the phosphorous acid, the phosphorus content of the organic phosphorus compound was 0.08% by weight, and the phosphorus content of the inorganic phosphorus compound was 3.00% by weight.

Thereafter, the reaction solution was treated at 40°C, at a nitrogen flow rate of $3.0 \times 10^{-3}$ m$^3$/kg·hr, and under a pressure of 665 Pa for 3 hours to remove the unreacted phosphorus trichloride, thereby obtaining the desired lauric acid chloride (542.1 g). In the lauric acid chloride obtained, the phosphorus content of the organic phosphorus compound was 0.20% by weight, and the phosphorus content of the inorganic phosphorus compound was 0.17% by weight.

(Comparative Example 3: Palm nucleus fatty acid chloride)

To palm nucleus fatty acid (400.0 g) was added dropwise 1.5/3 equivalent of phosphorus trichloride (130.0 g) at from 50 to 60°C to perform a chlorination reaction. After standing still for 2 hours to separate layers, phosphorous acid (57.0 g) of the lower layer was removed to obtain palm nucleus fatty acid chloride (472.5 g). In the reaction solution after removing the phosphorous acid, the phosphorus content of the organic phosphorus compound was 0.02% by weight, and the phosphorus content of the inorganic phosphorus compound was 1.0% by weight.

Thereafter, the reaction solution was treated at 60°C, at a nitrogen flow rate of $3.0 \times 10^{-3}$ m$^3$/kg·hr, and under a pressure of $200 \times 10^2$ Pa for 25 hours to remove the unreacted phosphorus trichloride, thereby obtaining palm nucleus fatty acid chloride (445.8 g). In the palm nucleus fatty acid chloride obtained, the phosphorus content of the organic phosphorus compound was 0.15% by weight, and the phosphorus content of the inorganic phosphorus compound was 0.02% by weight.

(Comparative Example 4: Palm oil fatty acid chloride)

To palm oil fatty acid (240.0 g) was added dropwise 0.9/3 equivalent of phosphorus trichloride (130.0 g) at from 50 to 60°C to perform a chlorination reaction. After standing still for 2 hours to separate layers, phosphorous acid (34.1 g) of the lower layer was removed to obtain palm oil fatty acid chloride (335.2 g). In the reaction solution after removing the phosphorous acid, the phosphorus content of the organic phosphorus compound was N. D. (less than 0.01% by weight), and the phosphorus content of the inorganic phosphorus compound was 0.4% by weight.

Thereafter, the reaction solution was treated at 30°C, at a nitrogen flow rate of $3.0 \times 10^{-3}$ m$^3$/kg·hr, and under a pressure of 665 Pa for 2 hours to remove the unreacted phosphorus trichloride. In the palm oil fatty acid chloride obtained, the phosphorus content of the organic phosphorus compound was 0.02% by weight, and the content of the inorganic phosphorus compound was 0.10% by weight.

(Comparative Example 5: Lauric acid chloride)

To lauric acid (400 g) was added dropwise 1.5/3 equivalent of phosphorus trichloride (130.0 g) at from 50 to 60°C to perform a chlorination reaction. After standing still for 2 hours to separate layers, phosphorous acid (56.9 g) of the lower layer was removed to obtain 474.6 g of lauric acid chloride. In the lauric acid chloride obtained, the phosphorus content of the organic phosphorus compound was 0.02% by weight, and the phosphorus content of the inorganic phosphorus compound was 1.00% by weight.

(Comparative Example 6: Stearic acid chloride)

To stearic acid (435.0 g) was added dropwise 2.0/3 equivalent of phosphorus trichloride (130.7 g) at from 60 to 65°C to perform a chlorination reaction. After standing still for 2 hours to separate layers, phosphorous acid (44.7 g) of the lower layer was removed to obtain a reaction solution (521.0 g). In the reaction solution after removing the phosphorous acid, the phosphorus content of the organic phosphorus compound was 0.05% by weight, and the phosphorus content of the inorganic phosphorus compound was 1.8% by weight.

Thereafter, the reaction solution was treated at 80°C, at a nitrogen flow rate of $3.0 \times 10^{-3}$ m$^3$/kg·hr, and under a pressure of 266.6 × 10 Pa for 1.5 hours to remove the unreacted phosphorus trichloride, thereby obtaining the desired stearic acid chloride (446.2 g). In the stearic acid chloride obtained, the phosphorus content of the organic phosphorus compound was 0.07% by weight, and the phosphorus content of the inorganic phosphorus compound was 0.02% by weight.

(Comparative Example 7: Palm oil fatty acid chloride)

To palm oil fatty acid (400.0 g) was added dropwise 1.5/3 equivalent of phosphorus trichloride (130.0 g) at from 50 to 60°C to perform a chlorination reaction. After standing still for 2 hours to separate layers, phosphorous acid (57.0 g) of the lower layer was removed to obtain a reaction solution (472.6 g). In the reaction solution after removing the phosphorous acid, the phosphorus content of the organic phosphorus compound was 0.02% by weight, and the phosphorus content of the inorganic phosphorus compound was 0.90% by weight.

Thereafter, the reaction solution was treated at 70°C, at a nitrogen flow rate of $3.0 \times 10^{-3}$ m$^3$/kg·hr, and under a pressure of $133.3 \times 10^{-1}$ Pa for 1 hour to remove the unreacted phosphorus trichloride, thereby obtaining the desired palm oil fatty acid chloride (452. 6 g). In the palm oil fatty acid chloride obtained, the phosphorus content of the organic phosphorus compound was 0.02% by weight, and the phosphorus content of the inorganic phosphorus compound was 0.01% by weight.

(Comparative Examples 8 to 14)

To the distilled palm oil fatty acid chloride of Comparative Example 1 were added phosphonic acid (special grade, produced by Wako Pure Chemical Industries, Ltd.) and dodecyl phosphate (produced by Wako Pure Chemical Industries, Ltd.), and the phosphorus contents of the inorganic phosphorus compound and the organic phosphorus compound were measured.

(Evaluation of turbidity of fatty acid chloride)

Each of the fatty acid chloride solutions obtained was put into a 100 ml sample bottle made of glass, observed its appearance at 25°C, and evaluated according to the criteria shown below.

○○:    Transparent
○:      Slightly turbid
∆:      Turbid
x:      Precipitated

(Evaluation of hue of fatty acid chloride (temporal stability))

Each of the fatty acid chlorides obtained was put into a 100 ml sample bottle made of glass, the bottle was lidded and stored at 25°C for one month, and change of hue (∆APHA) at 25°C was evaluated.

$$\text{∆APHA = (APHA value after temporal stability test) - (APHA}$$

$$\text{value before temporal stability test)}$$

○○: ∆APHA is from 0 to 29

○: ∆APHA is from 30 to 59

∆: APHA is from 60 to 89

✕: ∆APHA is 90 or more

[Table 1]

Table 1

|  | Example 1 | Example 2 | Example 3 | Example 4 |
|---|---|---|---|---|
| Charging Ratio of Phosphorus Trichloride | 1.5/3 | 1.5/3 | 1.8/3 | 2.0/3 |
| Phosphorus Content of Organic Phosphorus Compound after Step 1 (% by weight) | 0.02 | 0.02 | 0.04 | 0.05 |
| Phosphorus Content of Inorganic Phosphorus Compound after Step 1 (% by weight) | 0.90 | 1.00 | 1.50 | 1.80 |
| Free Fatty Acid after Step 1 (% by weight) | 1.5 | 1.5 | 1.4 | 1.3 |
| Phosphorus Content of Organic Phosphorus Compound after Step 2 (% by weight) | 0.06 | 0.10 | 0.10 | 0.08 |
| Phosphorus Content of Inorganic Phosphorus Compound after Step 2 (% by weight) | 0.15 | 0.10 | 0.30 | 0.11 |
| Free Fatty Acid after Step 2 (% by weight) | 1.6 | 1.4 | 0.8 | 1.5 |
| Turbidity | ○○ | ○ | ○ | ○○ |
| Hue (lapse of time) | APHA Value before Temporal Stability Test | 40 | 60 | 20 | 60 |
|  | APHA Value after Temporal Stability Test | 60 | 85 | 30 | 80 |
|  | Evaluation | ○○ | ○○ | ○○ | ○○ |

[Table 2]

Table 2

|  | Example 5 | Example 6 | Example 7 |
|---|---|---|---|
| Charging Ratio of Phosphorus Trichloride | 1.3/3 | 1.5/3 | 2.0/3 |
| Phosphorus Content of Organic Phosphorus Compound after Step 1 (% by weight) | 0.02 | 0.02 | 0.05 |
| Phosphorus Content of Inorganic Phosphorus Compound after Step 1 (% by weight) | 0.62 | 1.00 | 1.80 |
| Free Fatty Acid after Step 1 (% by weight) | 1.8 | 1.5 | 1.3 |
| Phosphorus Content of Organic Phosphorus Compound after Step 2 (% by weight) | 0.05 | 0.08 | 0.07 |
| Phosphorus Content of Inorganic Phosphorus Compound after Step 2 (% by weight) | 0.11 | 0.08 | 0.08 |
| Free Fatty acid after Step 2 (% by weight) | 1.9 | 1.3 | 1.4 |
| Turbidity | ○ | ○ | ○○ |
| Hue (lapse of time) | APHA Value before Temporal Stability Test | 60 | 50 | 50 |
|  | APHA Value after Temporal Stability Test | 80 | 65 | 60 |
|  | Evaluation | ○○ | ○○ | ○○ |

[Table 3]

Table 3

| | Comparative Example 1 | Comparative Example 2 | Comparative Example 3 | Comparative Example 4 |
|---|---|---|---|---|
| Charging Ratio of Phosphorus Trichloride | 1.5/3 | 2.5/3 | 1.5/3 | 0.9/3 |
| Phosphorus Content of Organic Phosphorus Compound after Step 1 (% by weight) | 0.02 | 0.08 | 0.02 | N. D. |
| Phosphorus Content of Inorganic Phosphorus Compound after Step 1 (% by weight) | 0.90 | 3.00 | 1.00 | 0.40 |
| Free Fatty Acid after Step 1 (% by weight) | 1.5 | 1.3 | 1.5 | 13.5 |
| Phosphorus Content of Organic Phosphorus Compound after Step 2 (% by weight) | 0.01 | 0.20 | 0.15 | 0.02 |
| Phosphorus Content of Inorganic Phosphorus Compound after Step 2 (% by weight) | N. D. | 0.17 | 0.02 | 0.10 |
| Free Fatty Acid after Step 2 (% by weight) | 0.4 | 1.5 | 1.2 | 13.1 |
| Turbidity | ○○ | Δ | ○ | ○○ |
| Hue (lapse of time) | APHA Value before Temporal Stability Test | 5 | 30 | 80 | 60 |
| | APHA Value after Temporal Stability Test | 100 | 70 | 160 | 130 |
| | Evaluation | × | ○ | Δ | Δ |

[Table 4]

Table 4

| | Comparative Example 5 | Comparative Example 6 | Comparative Example 7 |
|---|---|---|---|
| Charging Ratio of Phosphorus Trichloride | 1.5/3 | 2.0/3 | 1.5/3 |
| Phosphorus Content of Organic Phosphorus Compound after Step 1 (% by weight) | 0.02 | 0.05 | 0.02 |
| Phosphorus Content of Inorganic Phosphorus Compound after Step 1 (% by weight) | 1.00 | 1.80 | 0.90 |
| Free Fatty Acid after Step 1 (% by weight) | 1.5 | 1.8 | 1.5 |
| Phosphorus Content of Organic Phosphorus Compound after Step 2 (% by weight) | -- | 0.07 | 0.03 |
| Phosphorus Content of Inorganic Phosphorus Compound after Step 2 (% by weight) | - | 0.02 | 0.01 |
| Free Fatty Acid after Step 2 (% by weight) | - | 1.5 | 1.3 |
| Turbidity | × | ○ | ○ |

(continued)

| | | Comparative Example 5 | Comparative Example 6 | Comparative Example 7 |
|---|---|---|---|---|
| Hue (lapse of time) | APHA Value before Temporal Stability Test | 30 | 30 | 20 |
| | APHA Value after Temporal Stability Test | 35 | 140 | 160 |
| | Evaluation | ○○ | × | × |

[Table 5]

Table 5

| | Comparative Example 8 | Comparative Example 9 | Comparative Example 10 | Comparative Example 11 | Comparative Example 12 | Comparative Example 13 | Comparative Example 14 |
|---|---|---|---|---|---|---|---|
| Phosphorus Content of Dodecyl Phosphate Added (% by weight) | 0.01 | 0.01 | 0.01 | 0.05 | 0.07 | 0.10 | 0.06 |
| Phosphorus Content of Phosphonic Acid Added (% by weight) | 0.05 | 0.10 | 0.30 | 0.00 | 0.00 | 0.00 | 0.15 |
| Phosphorus Content of Organic Phosphorus Compound (% by weight) | 0.01 | 0.01 | 0.01 | 0.05 | 0.07 | 0.10 | 0.06 |
| Phosphorus Content of Inorganic Phosphorus Compound (% by weight) | 0.10 | 0.05 | 0.30 | N. D. | N.D. | N.D. | 0.15 |
| Turbidity | ○○ | ○ | △ | ○○ | ○○ | ○ | ○ |
| Hue (lapse of time) | × | × | △ | × | △ | △ | △ |

In the examples shown in Tables 1 to 2, the turbidity of the fatty acid chloride is small and the change of hue due to the lapse of time is inhibited.

In Comparative Example 1 shown in Table 3, the phosphorus compound has been evaporated from the fatty acid chloride by a distiller, the phosphorus content of the inorganic phosphorus compound and the phosphorus content of the organic phosphorus compound are small, and the change of hue due to the lapse of time is large.

In Comparative Example 2, the equivalent of phosphorus trichloride is large, the phosphorus content of the organic phosphorus compound is large, and the turbidity is recognized.

In Comparative Example 3, the pressure in Step 2 is high, the phosphorus content of the inorganic phosphorus compound is small, the phosphorus content of the organic phosphorus compound is large, and the change of hue due to the lapse of time is large.

In Comparative Example 4, the equivalent of phosphorus trichloride is small, the phosphorus content of the organic phosphorus compound is small, and the change of hue due to the lapse of time is large.

In Comparative Example 5 shown in Table 4, Step 2 is not performed, the phosphorus content of the inorganic phosphorus compound is large, the phosphorus content of the organic phosphorus compound is small, and the precipitates are observed.

In Comparative Example 6, the temperature in Step 2 is high, the phosphorus content of the inorganic phosphorus compound is small, and the change of hue due to the lapse of time is large.

In Comparative Example 7, the temperature in Step 2 is high, the phosphorus content of the inorganic phosphorus compound and the phosphorus content of the organic phosphorus compound are small, and the change of hue due to the lapse of time is large.

In Comparative Examples 8 to 14 shown in Table 5, the phosphorus content of the inorganic phosphorus compound and the phosphorus content of the organic phosphorus compound are adjusted by adding the inorganic phosphorus compound or the organic phosphorus compound from outside to the furry acid chloride of Comparative Example 1 in which the phosphorus has been removed by distillation.

In Comparative Examples 8 to 10, the phosphorus content of the organic phosphorus compound is small, and the change of hue due to the lapse of time is large.

In Comparative Examples 11 to 13, the phosphorus content of the inorganic phosphorus compound is small, and the change of hue due to the lapse of time is large.

In Comparative Example 14, although both the phosphorus content of the inorganic phosphorus compound and the phosphorus content of the organic phosphorus compound are not different from those of the examples according to the invention, the change of hue due to the lapse of time is large.

Although the invention has been described in detail and by reference to specific embodiments, it is apparent to those skilled in the art that it is possible to add various alterations and modifications insofar as the alterations and modifications do not deviate from the spirit and the scope of the invention.

This application is based on a Japanese patent application filed on April 14, 2014 (Japanese Patent Application No. 2014-82538), and the contents of which are incorporated herein by reference. Also, all the references cited herein are incorporated as a whole.

**Claims**

1. A production method of a fatty acid chloride comprising: performing Step 1 and Step 2 in this order:

   Step 1: a step of reacting a fatty acid having from 8 to 22 carbon atoms with from 1/3 to 2/3 equivalent of phosphorus trichloride relative to the fatty acid to prepare a fatty acid chloride, and removing phosphorous acid which is a by-product to obtain a reaction product;
   Step 2: a step of treating the reaction product obtained in the Step 1 at a temperature from 10 to 60°C, at a nitrogen flow rate from $1.0 \times 10^{-4}$ to $1.0 \times 10^{-2}$ m$^3$/kg·hr, and under a pressure from $133.3 \times 10^{-2}$ to $133.3 \times 10^{2}$ Pa so as to react an unreacted phosphorus trichloride with the fatty acid and the fatty acid chloride to prepare an organic phosphorus compound and so as to be distilled off the unreacted phosphorus trichloride, thereby obtaining a fatty acid chloride.

2. The method as claimed in Claim 1, wherein a phosphorus content of an inorganic phosphorus compound contained in the fatty acid chloride obtained in the Step 2 is from 0.03 to 0.3% by weight, and a phosphorus content of the organic phosphorus compound contained in the fatty acid chloride obtained in the Step 2 is from 0.04 to 0.1% by weight.

3. A fatty acid chloride obtained by the method as claimed in Claim 1 or 2.

## INTERNATIONAL SEARCH REPORT

| | International application No. |
|---|---|
| | PCT/JP2015/061503 |

### A. CLASSIFICATION OF SUBJECT MATTER

*C07C51/60*(2006.01)i, *C07C53/42*(2006.01)i, *C07C57/66*(2006.01)i

According to International Patent Classification (IPC) or to both national classification and IPC

### B. FIELDS SEARCHED

Minimum documentation searched (classification system followed by classification symbols)
C07C51/60, C07C53/42, C07C57/66

Documentation searched other than minimum documentation to the extent that such documents are included in the fields searched
Jitsuyo Shinan Koho       1922–1996   Jitsuyo Shinan Toroku Koho   1996–2015
Kokai Jitsuyo Shinan Koho  1971–2015   Toroku Jitsuyo Shinan Koho   1994–2015

Electronic data base consulted during the international search (name of data base and, where practicable, search terms used)
CAplus/REGISTRY/CASREACT(STN)

### C. DOCUMENTS CONSIDERED TO BE RELEVANT

| Category* | Citation of document, with indication, where appropriate, of the relevant passages | Relevant to claim No. |
|---|---|---|
| X | JP 1-211547 A  (NOF Corp.), 24 August 1989 (24.08.1989), page 3, lower left column, lines 1 to 13; tables 1 to 3 (Family: none) | 1-3 |
| X A | JP 63-316753 A  (BASF AG.), 26 December 1988 (26.12.1988), page 3, lower right column, lines 4 to 14; page 4, upper left column, lines 8 to 18; page 4, upper right column, line 12 to lower left column, line 10 & US 4900479 A          & EP 296404 A2 | 3 1,2 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered  to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 16 June 2015 (16.06.15) | 30 June 2015 (30.06.15) |

| Name and mailing address of the ISA/ Japan Patent Office 3-4-3,Kasumigaseki,Chiyoda-ku, Tokyo 100-8915,Japan | Authorized officer |
|---|---|
| | Telephone No. |

Form PCT/ISA/210 (second sheet) (July 2009)

**INTERNATIONAL SEARCH REPORT**

| International application No. |
| --- |
| PCT/JP2015/061503 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2014-58458 A (NOF Corp.), 03 April 2014 (03.04.2014), paragraphs [0020] to [0041] (Family: none) | 1-3 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 11255703 A
- JP 6041000 A
- JP 2014082538 A